Europäisches Patentamt

European Patent Office

Office européen des brevets

(19)

(11) Publication number: **0 164 190**
A2

# (12) EUROPEAN PATENT APPLICATION

(21) Application number: 85302445.3

(22) Date of filing: 04.04.85

(51) Int. Cl.⁴: **A 61 L 2/00,** C 08 L 23/10, C 08 L 23/16, C 08 L 23/06, C 08 J 5/18 // (C08L23/10, 23:16, 23:06)

(30) Priority: 05.04.84 US 597015

(43) Date of publication of application: 11.12.85 Bulletin 85/50

(84) Designated Contracting States: **AT BE CH DE FR GB IT LI NL SE**

(71) Applicant: **Exxon Research and Engineering Company, P.O.Box 390 180 Park Avenue, Florham Park New Jersey 07932 (US)**

(72) Inventor: **Hazelton, Donald Ross, 89 Glenmore Drive, Chatham New Jersey (US)**
Inventor: **Jansen, Deborah Stamp, 10 Victoria Drive, Annandale New Jersey (US)**
Inventor: **Puydak, Robert Chester, 17 John Adams Court, Cranbury New Jersey (US)**

(74) Representative: **Dew, Melvyn John et al, Esso Chemical Ltd. Esso Chemical Research Centre P.O. Box 1, Abingdon Oxfordshire, OX13 6BB (GB)**

(54) Sterilizable packages and processes.

(57) Sterilizable packages can be prepared from a blend of polypropylene, ethylene-propylene elastomer and polyethylene. The packages can be sterilized by ethylene oxide, gamma radiation and steam.

EP 0 164 190 A2

STERILIZABLE PACKAGES AND PROCESSES

This invention relates to plastic packages and containers that can be heat sealed and sterilized and to the use of certain films in the production of such articles. There is a need for packages and containers that have adequate physical properties and yet can be closed by heat sealing and withstand sterilization by all of the conventional methods including ethylene oxide, steam and gamma radiation. It can also be advantageous to form such packages and containers from thermoplastic resin materials that are in the form of films that can be made on processing equipment that is set up to form easily processed conventional low density polyethylene resins.

The articles of this invention are heat sealable and sterilizable packages and containers that are made from selected triblend resin compositions - a blend of a first member selected from polypropylene resins, a second member selected from ethylene propylene elastomers and a third member selected from ethylene resins. The resins may be homopolymers or copolymers as hereafter discussed. The proportions are selected to give a balance of physical properties and good heat stability. These compositions can be formed into shapes for making packaging, e.g., films of up to about 10 mils thickness, sheets of about 10 to 100 mils in thickness. The package material can be formed using equipment commonly used to form polyolefin film of conventional polyethylene types. Preparation of film is performed at temperatures higher than those used in the conventional polyethylene types. Thermoforming is accomplished in a similar manner. Packages and containers are fabricated from the films or powders or pellet form of the resin composition. One construction has a composite article which employs the resin composition as an inner wrap for the sterilizable contents of the package; other constructions utilize the compositions as the principal material.

-2-

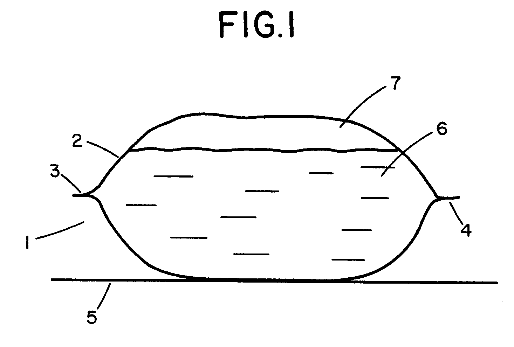

With regard to the accompanying drawings, Figure 1 is an illustration of a sterilizable package having a heat sealed closure which is formed according to this invention. Figure II is an illustration of a sterilizable thermoformed package according to this invention.

The packages and containers of this invention and the fabrication, heat sealing and sterilization thereof are achieved by making use of a selected triblend resin composition. The components of the triblend thermoplastic resin film are a first member selected from polypropylene resin, a second member selected from ethylene propylene elastomers and a third member selected from polyethylene resins. The first member is preferably a thermoplastic crystalline (usually isotactic) polypropylene resin that has a chemical composition of essentially monomeric propylene units or propylene units together with a relatively small proportion of co-monomer such as ethylene or butene. The copolymers are known as reactor copolymers, random or block. These materials are stereoregular configurations with a melt flow range preferably 0.5 to 20, particularly about 7.5. Their densities are preferably 0.89 to 0.91 g/cc and and they may have crystalline melting points in the region of $120° - 150° C$. These materials are an article of commerce and are available as powders, pellets and the like.

The second member is an ethylene-propylene elastomer which is either a copolymer of ethylene and propylene or a terpolymer of ethylene and propylene with a small proportion of termonomer such as ethylidene norborene or 1,4 hexadiene or dicyclopentadiene. It preferably has a Mooney viscosity in the range of 15 to 70 at $260° F$, $M_{L(1+8)}$ and like polypropylene is an article of commerce.

The third member is a polyethylene resin. Its chemical composition may be a homopolymer of ethylene or a copolymer of ethylene and a comonomer of the acetate or acrylate family. The ethylene content is preferably in the range of 70 to 100% by total weight of resin. The co- monomer is present in amounts preferably up to 30 mole %. The polyethylene resins may be the traditional high pressure variety polymer, having a density in the range of about .91 to .94 g/cc and a melt flow index of about 0.2 to 20. It may also be a linear low pressure variety having a density in the range of about .92 to .94 and a melt flow index of about 0.2 to 20. It may also be a high density variety having a density in the range of about .94 to .965 and a melt flow index in the range of .05 to 20. The crystalline melting points for these resins are preferably from 80° to 120° C. All of these resins are articles of commerce.

The proportions of the members are by weight at least 20% by weight of the first member, 10 to 60% by weight of the second member and 10 to 60% by weight of the third member. Especially preferred compositions have proportions in the range of about 30 to 70 weight % for the first member, 20 to 50 weight % for the second member, and 10 to 50 weight % for the third member.

The resin composition is prepared by blending the members together under high shear to form a molten mixture which is then cooled to form a powder or pellet. In morphological terms, the blend has the first member as a continuous phase with the second member providing compatibility to the third member which allows the blend to have desirable physical properties and good processability for forming packages and articles. This morphological structure also contributes to the steam sterilizability and gamma radiation resistance of the material. At lesser proportions of polypropylene, gamma resistance is retained but steam sterilizabilituy and high temperature dimensional stability are sacrificed.

The triblend resin can then be converted into films for preparation of package components. It is readily formed into films by using conventional low pressure polyethylene film blowing equipment. This process is one where triblend resin compositions are melted in an extruder and then blown into a bubble to form a film which is cooled, slit and rolled onto a spool. It is remarkable that with such large proportions of polypropylene present, the film can be made in this manner.

It is noteworthy that the resin composition, during melting and shaping exhibits a trait known as "melt strength". By this, both film and sheet can readily be produced in thicknesses ranging from about 0.25 mils to about 35 mils. Moreover, this property lends itself to other plastic shaping techniques to form packages and containers, such as blow molding, injection molding, casting and thermoforming.

The films are characterized by having a combination of physical properties that satisfies a balance of end-use objectives. These are tensile strength, elongation at break, stiffness, tear strength and puncture impact strength. In addition, the films are readily heat sealable and can be subjected to sterilization by heat, ethylene oxide or gamma (cobalt) radiation. In packaging, the film can itself be used as the wrap to enclose an article, it can also be one of several wrappings as is usual in sterilized medical packages. The film can be laminated to other bases to yield a composite packaging material. In terms of structure, the container or package can have at least one major element made from the triblend resin compositions. In blow molded/injection molded bottles or pouches, the entire article can be formed from the resin material because it is adapted to both blow molding and injection molding. The resin compositions are especially useful for the elements of the container that are adjacent to the contents; in these cases the heat sealability and sterilizability of the composition is used to maximum advantage. Bags can be made by slitting the film and then heat sealing the edges to form pouches. These are filled, closed by heat sealing and sterilized by steam or radiation. This filled package can then be inserted into an outer container such as cardboard for shipping. Where ethylene oxide is the sterilization media, the container or package will need a paper lid or section that is permeable to the ethylene oxide.

Because film is a basic packaging material, processability of the resin compositions into film is a useful advantage of this invention. Moreover, the film can be heat sealed to other materials to make package windows or package walls. Vicat softening points for the resin composition can be controlled by the proportion of ingredients. These can be varied from about 62°C to about 130°C by increasing the proportion of the first member. The ability to achieve low softening points indicates ease of controlling heat sealability for fabrication of articles according to this invention. The range of practical temperatures for heat sealibility is of importance because the films and sheets of this invention can achieve good seal strengths over a wide range of temperatures to accomodate equipment variations. The polypropylene first member links good mechanical properties to the material while the ethylene-propylene rubber second member and polyethylene third member give the low temperature sealing capability. As a whole, the material resists thinning during heat sealing.

To illustrate the invention, film samples are prepared by blending components as described above and then processing molten resin compositions on film blowing equipment. The equipment comprises an extruder, a die, a film blowing unit, and a nip roller. The triblend resin composition has 50% by weight of the first member, 30% by weight of the second member and 20% by weight of the third member. In this example, the first member is polypropylene homopolymer of 0.904 g/cc density; the second member is an ethylene propylene elastomer having a viscosity of $M_{L(1+8)}$ at 250 °F of 45 and the third member is a low density polyethylene resin of 0.902 g/cc density. The film gauge as well as the draw down (DDR) and blow up (BUR) ratios

## TABLE I

| Gauge, mils | 3.8 | 3.0 | 2.2 | 1.0 | 0.3 |
|---|---|---|---|---|---|
| BUR | 3.0:1 | 2.9:1 | 2.9:1 | 2.9:1 | 2.8:1 |
| DUR | 1.8:1 | 2.4:1 | 3.3:1 | 7.1:1 | 25.0:1 |
| Tensile Strength, psi | | | | | |
| MD | 3,490 | 4,480 | 4,600 | 3,970 | 4,830 |
| TD | 3,960 | 4,150 | 3,410 | 3,290 | 4,120 |
| Elongation, % | | | | | |
| MD | 875 | 945 | 810 | 525 | 195 |
| TD | 1,020 | 930 | 645 | 545 | 160 |
| 1% Secant Modulus, psi | | | | | |
| MD | 34,800 | 37,200 | 37,400 | 43,700 | 17,700 |
| TD | 42,000 | 32,200 | 41,200 | 36,300 | 26,800 |
| Elmendorf Tear, g/mil | | | | | |
| MD | 98 | 76 | 93 | 107 | 8 |
| TD | 88 | 90 | 112 | 353 | 517 |

are shown in Table I. The measurement of physical properties indicated above is based upon accepted industry standards, as specified hereinafter.

A particular application of the invention is directed towards packaging where the advantageous heat sealing and sterilizability of the films can be seen to advantage. Figure 1 illustrates an arrangement for such packaging. In Figure 1, a cross-sectional illustration of a package construction is given. There is a package (1) which is the container (2) and its contents (6). The container illustrated is a pouch having a first edge (3) and a second edge (4) formed by heat sealing together the film of the container. In the filled closed container, the remainder of the edges would be sealed. Preferably, at least one edge is an opening which after filling is itself heat sealed. In a preferred construction, there is also an overwrap (5). This is desirably an outer covering for the inner package. The contents can have a liquid or solid portion (6) and/or a vapor portion (7). Especially, the contents may be materials which require sterilization for use in medical applications.

The sterilization in this example can be accomplished by heat or radiation. Where radiation is used, the dosage may be up to 5.0 megarads in strength. Radiation is more common for sterilization of solids, e.g., syringes, sutures and gauze. For liquids, steam sterilization is preferred so that adverse chemical side reactions of the contents can be avoided.

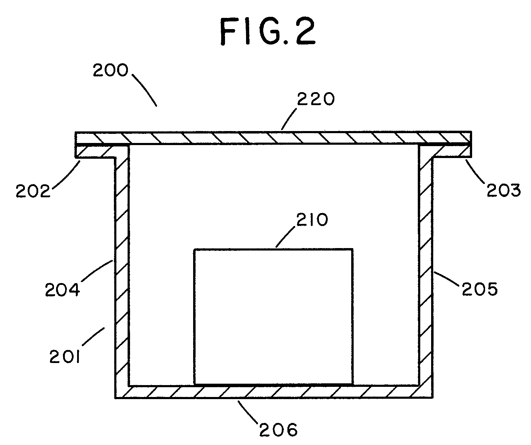

Packages formed from sheets are illustrated in Figure 2. In this illustration, the package (200) is made from a tub (201) and a lid (220). The tub is prepared by deep draw thermoforming of a sheet of the resin material described in this invention. It has a continuous cross-section. There are edge lips (202), (203), which join sidewalls (204) and (205) that connect to a bottom (206). The draw ratio is relatively high, i.e., the ratio of depth to width is high. The contents are a solid and shown as (210).

Two features of the invention apply to constructions illustrated in Figure 2. The container is sterilizable and the wall thickness is relatively uniform so that thin sections in walls or corners are avoided.

The lid may be opaque paper that is heat sealed to the tub. The contents are sterilized by gamma radiation. It may also be advantageous to overwrap the package as was explained in reference to Figure 1.

The packages and containers of this invention can also be used for "hot-fill materials". In this procedure, the fill material is at a temperature sufficient to sterilize or maintain the film in a wholesome condition. It is packaged while hot and then stored. Because of the thermal stability of the containers made from the resin compositions of this invention, the container will retain its physical dimensions while the heated contents cool. Foodstuffs are principal examples, especially brine packed foods.

A series of film samples are prepared to illustrate the application of the invention to structures such as shown in Figures 1 and 2. Packaging for medical applications presents a very demanding set of objectives with minimum tradeoffs available between contrasting objectives. The following examples also illustrate other methods for preparing containers according to this invention and the several preferred species for practice of the invention.

Resin formulations are prepared by blending together the first member, the second member and the third member in a Banbury mixer. After becoming molten, the mixture is shaped into pellets. The pellets are re-melted and can be extruded into a film by casting or blowing. Sheet can be formed by calendering. The film is then formed into a pouch by heat sealing edge portions, filling and heat sealing the opening. It is covered with an overwrap and then it is sterilized at about 115° to 125°C with steam for about 25 - 45 minutes.

Table II illustrates the change in haze from autoclaving when a film sample of approximately 7 mils is sterilized. The ratio of resin components are 50 weight % first member, 30 weight % second member, 20 weight % third member. The films are generally autoclave stable.

### TABLE II

% Haze

| | | | | | |
|---|---|---|---|---|---|
| Before Autoclave | 39.6 | 40.6 | 40.9 | 33.2 | 33.3 |
| After | 47.9 | 44.2 | 51.0 | 43.4 | 39.9 |
| $\Delta$ | +8.3 | +3.6 | +10.1 | +10.2 | +6.6 |

In Table III, the samples illustrate formulations where auxiliary additives have been used to improve haze as well as variations in the type of polyethylene used as the third member. The polyethylene types are low density polyethylene resin (X-1), ethylene-vinyl acetate copolymer (5% VA) (X-2); ethylene vinyl acetate copolymer (18% VA) (X-3); ethylene methyl acrylate copolymer (20% by wt., MA) (X-4). The second member is an ethylene-propylene elastomer. Type 1 has an $M_{L(1+8)}$ of 50; Type 2 has an $M_{L(1+8)}$ of 22. The additives are for processing, stability and haze control. In addition, these affect stiffness.

For optimum resistance to haze increase, it is preferred to use low molecular weight polyethylene wax as an additive. Antioxidants also tend to reduce haze increase.

In the forming of the film by casting, it is also desirable to minimize melt temperature for optimizing haze.

A film sample is prepared from:

45 pts - first member - polypropylene resin reactor copolymer

15 pts - second member - ethylene-propylene elastomer

40 pts - third member - polyethylene resin, and

0.3 pts - hydrolysis control agent

0.2 pts - slip agent.

The above composition is preferred for film that is made into sterilizable bags for solutions as described

TABLE III

| | A | B | C | D | E | F | G | H | I | J | K | L |
|---|---|---|---|---|---|---|---|---|---|---|---|---|
| First Member | 40 | 40 | 40 | 40 | 40 | 40 | 40 | 40 | 40 | 40 | 40 | 40 |
| Third Member | | | | | | | | | | | | |
| X-1 | 25 | - | - | - | - | - | - | - | - | - | - | - |
| X-2 | - | 25 | - | - | - | - | - | - | - | - | - | 30 |
| X-3 | - | - | 25 | - | - | - | - | 20 | 30 | 30 | 30 | - |
| X-4 | - | - | - | 25 | - | - | - | - | - | - | - | - |
| Second Member | | | | | | | | | | | | |
| Type I | 35 | 35 | 35 | 35 | 35 | - | - | 25 | 25 | 15 | 25 | 25 |
| Type II | - | - | - | - | - | 35 | 60 | - | - | - | - | - |
| Primol 355 | - | - | - | - | 25 | 25 | - | 15 | 5 | 5 | - | 5 |
| Irganox D225 | 0.15 | 0.15 | 0.15 | 0.15 | 0.15 | 0.15 | 0.15 | 0.15 | 0.15 | 0.15 | 0.15 | 0.15 |
| VISTANEX L -120 | - | - | - | - | - | - | - | - | - | 10 | - | - |
| AC 9 PE | - | - | - | - | - | - | - | - | - | - | 5 | - |
| Secant Flex Modulus, | | | | | | | | | | | | |
| psi | 56,400 | 56,300 | 53,800 | 53,700 | 26,100 | 24,100 | 37,000 | 31,000 | 38,000 | 36,900 | 48,800 | 43,000 |
| | | | | | Greasy | Greasy | Greasy | Sl. Greasy | | | | |
| Gauge, mils | 8.2 | 8.2 | 8.0 | 7.8 | 7.3 | 7.6 | 8.3 | 8.6 | 8.4 | 8.7 | 6.6 | 8.7 |
| Haze, % | | | | | | | | | | | | |
| Before Autoclave | 30 | 35 | 34 | 28 | 28 | 49 | 56 | 38 | 34.5 | 43.8 | 30 | 40 |
| After | 56 | 51 | 42 | 41 | 54 | 63 | 61 | 47 | 44 | 47 | 32 | 46.6 |
| Δ | +26 | +16 | +8 | +13 | +26 | +14 | +5 | +9 | +9.5 | +3.2 | +2 | +6.6 |
| Colorimeter Readings | | | | | | | | | | | | |
| Yellowness Before | 2.5 | 3.86 | 2.28 | 2.34 | 2.80 | 3.12 | 2.97 | 2.36 | 2.28 | 2.37 | 1.98 | 2.67 |
| Index  After | 5.5 | 4.86 | 3.14 | 3.02 | 5.35 | 5.20 | 6.40 | 4.47 | 4.54 | 3.89 | 3.47 | 5.28 |
| Δ | +3.0 | +1.0 | +0.8 | +0.7 | +2.6 | +2.1 | +3.4 | +2.1 | +2.2 | +1.5 | +1.5 | +2.6 |
| L  Before | 95.7 | 92.91 | 95.29 | 95.4 | 94.87 | 93.26 | 93.96 | 95.49 | 94.98 | 94.71 | 95.27 | 95.0 |
| After | 91.0 | 93.45 | 94.56 | 94.09 | 92.84 | 89.5 | 92.13 | 91.95 | 91.86 | 93.34 | 93.15 | 93.53 |
| b  Before | +1.28 | +1.96 | +1.15 | +1.22 | +1.51 | +1.44 | +1.48 | +1.22 | +1.15 | +1.15 | +0.99 | +1.39 |
| After | +2.79 | +2.52 | +1.64 | +1.57 | +2.80 | +2.41 | +3.18 | +2.18 | +2.20 | +1.64 | +1.72 | +2.66 |
| Δ | +1.5 | +0.5 | +0.4 | +0.4 | +1.3 | +1.0 | +1.7 | +1.0 | +1.0 | +0.4 | +0.7 | +1.3 |

above. The wax additives can be AC-9 polyethylene wax or AC-316 oxidized polyethylene wax. The hydrolysis control agent can be Ethyl Antioxidant 330, see U.S.P. 4,140,162. The slip agent can be Armoslip O slip agent.

Articles can also be fabricated by thermoforming the triblend resin compositions into trays, tubs and the like. These articles may be subjected to sterilization by radiation at dosages of 2.5 and 5.0 megarads, or alternatively by steam at 125°C for about thirty minutes. The physical properties of these sterilized articles will be about 70% of the values for the non-sterilized articles. It is noted that in respect to the second member, the resistance to change in physical properties increases as the ethylene content is increased. Especially, in that polypropylene is known to show severe post irradiation embrittlement, it is surprising that an article with the significant levels of polypropylene resin disclosed herein has retained significant physical properties so that the article is still useful as a package or container.

The following examples of packages made by thermoforming exemplify the versatility of sterilizability, good processing and retention of physical properties which are features of this invention.

Sheets are made from resin compositions having (A) 65% by weight of the first member, 21% by weight of the second member and 14% by weight of the third member and (B) 61% by weight of the first member, 25% by weight of the second member and 14% by weight of the third member. Antioxidants and stabilizers are included.

The resin mixture is cast into sheets and this is thermoformed into rectangular tubs of about 4" x 4" x 1-1/4" depth. The wall thickness is in the range of 20 - 25 mils. The samples are subjected to steam autoclaving of 30 minutes at 250°F. Other samples are sterilized with gamma radiation at 2.5 and 5.0 megarads.

Physical properties as measured by accelerated aging test, 120°F in hot air, illustrate the physical properties

of the samples:

## TABLE IV

### SAMPLE A - AUTOCLAVE STERILIZATION

| | AGED, WEEKS | | | |
|---|---|---|---|---|
| | 0 | 2 | 4 | 8 |
| Tensile Strength psi | | | | |
| @ Yield | 3760 | 2980 | 3085 | 3245 |
| @ Break | 3110 | 2450 | 2580 | 2635 |
| Elongation % | | | | |
| @ Yield | 19 | 16 | 16 | 15 |
| @ Break | 505 | 420 | 760 | 405 |
| Puncture Impact in lbs/mil | 0.8 | 1.6 | 1.5 | 2.0 |
| "b" Gardner Color | 7.4 | 7.5 | 7.1 | 9.6 |

## TABLE V

### SAMPLE B - AUTOCLAVE STERILIZATION

| | AGED, WEEKS | | | |
|---|---|---|---|---|
| | 0 | 2 | 4 | 8 |
| Tensile Strength psi | | | | |
| @ Yield | 3420 | 3155 | 3215 | 3220 |
| @ Break | 3295 | 2750 | 2995 | 2780 |
| Elongation % | | | | |
| @ Yield | 12 | 19 | 19 | 18 |
| @ Break | 960 | 1115 | 1025 | 1000 |
| Puncture Impact in lbs/mil | 1.0 | 1.5 | 1.5 | 1.6 |
| "b" Gardner Color | 10.0 | 11.7 | 9.3 | 13.1 |

## TABLE VI

### IRRADIATION

#### SAMPLE A - 2.5 M Rads

|  | AGED, WEEKS | | | |
| --- | --- | --- | --- | --- |
|  | 0 | 2 | 4 | 8 |
| Tensile Strength psi | | | | |
| @ Yield | 2890 | 2660 | 2880 | 2830 |
| @ Break | 3360 | 2990 | 3675 | 3505 |
| Elongation, % | | | | |
| @ Yield | 13 | 12 | 13 | 15 |
| @ Break | 1215 | 1210 | 1635 | 1450 |
| Puncture Impact, in lbs/mil | 2.5 | 2.2 | 1.7 | 2.3 |
| "b" Gardner Color | 11.2 | 12.7 | 13.1 | 14.6 |

#### SAMPLE A - 5 M Rads

|  | AGED, WEEKS | | | |
| --- | --- | --- | --- | --- |
|  | 0 | 2 | 4 | 8 |
| Tensile Strength psi | | | | |
| @ Yield | 2930 | 2960 | 2775 | 3065 |
| @ Break | 3790 | 2670 | 3090 | 2960 |
| Elongation, % | | | | |
| @ Yield | 10 | 14 | 15 | 15 |
| @ Break | 1590 | 1280 | 1300 | 1180 |
| Puncture Impact in lbs/mil | 1.4 | 2.5 | 1.6 | 2.0 |
| "b" Gardner Color | 12.4 | 13.5 | 14.4 | 16.4 |

0164190

-14-

## TABLE VII

### IRRADIATION

#### SAMPLE B - 2.5 M Rads

|  | AGED, WEEKS | | | |
|---|---|---|---|---|
|  | 0 | 2 | 4 | 8 |
| Tensile Strength psi | | | | |
| @ Yield | 3090 | 2910 | 3035 | 3000 |
| @ Break | 3790 | 3815 | 3700 | 3615 |
| Elongation, % | | | | |
| @ Yield | 12 | 15 | 16 | 15 |
| @ Break | 1430 | 1315 | 1565 | 1445 |
| Puncture Impact | | | | |
| in lbs/mil | 1.3 | 1.7 | 1.7 | 1.7 |
| "b" Gardner Color | 13.7 | 14.7 | 18.9 | 16.1 |

#### SAMPLE B - 5 M Rads

|  | AGED, WEEKS | | | |
|---|---|---|---|---|
|  | 0 | 2 | 4 | 8 |
| Tensile Strength psi | | | | |
| @ Yield | 3940 | 2990 | 2940 | 2330 |
| @ Break | 2470 | 2315 | 2310 | 2445 |
| Elongation, % | | | | |
| @ Yield | 12 | 14 | 15 | 16 |
| @ Break | 1520 | 780 | 780 | 1080 |
| Puncture Impact | | | | |
| in lbs/mil | 1.0 | 1.5 | 1.4 | 1.5 |
| "b" Gardner Color | 12.8 | 13.2 | 13.4 | 17.1 |

Samples on 6 month shelf life tests exhibit the same physical property relationships.

From the foregoing it can be seen that this invention provides substantially improved film components that have combinations of stiffness, heat sealability and resistance to puncture and haze development that are needed for sterilizable medicinal packaging. Also, the advantages of such packaging are related to the interaction of the resin components as well as the preferred additives discussed above. While the invention has been described to illustrate preferred embodiments, it is equally within the scope of this invention to practice embodiments common to the art such as interchanging contents of the packages where instruments are substituted for medical solutions, or where film is prepared in multi-ply or laminate form rather than single-ply form.

The above physical properties were measured in accordance with the following industrial standards.

| Property | ASTM Test |
| --- | --- |
| Blend melt flow rate | D 1238 (L) |
| Tensile strength | D 882 |
| Elongation % | D 882 |
| 1% secant modulus (stiffness) | D 882 |
| Tear strength (Elmendorf) | D 1922 |
| Dart drop impact | D 1709 |
| Puncture impact ... 5 in/min rate | |
| Haze | D 1003 |
| Gloss 45$^{\circ}$ | D 2457 |

CLAIMS:

1. The use in the production of sterilizable articles or components thereof, of a heat sealable thermoplastic film comprising a compatible blend of

(a) at least 20 wt% of a polypropylene resin

(b) from 10 to 60 wt% of an ethylene propylene elastomer and

(c) from 10 to 60 wt% of a polyethylene resin, which film has a thickness of from 0.25 to 35 mils (0.000635 to 0.0889 cm), a tensile strength of from 2000 to 7000 psi (13.79 to 48.265 MPa), an elongation at break of at least 400%, a stiffness of from 15000 to 100000 psi (103.42 - 689.50 MPa), and a tear strength measured in the machine direction of from 40 to 600 g/mil (15748 to 236220 g/cm).

2. The use according to claim 1 wherein the film comprises from 30 to 70 wt% (a), from 20 to 50 wt% (b) and from 10 to 50 wt% (c).

3. The use according to claim 1 or 2 wherein in the film blend (a) comprises reactor copolymer and/or (b) comprises a terpolymer and/or (c) comprises homopolyethylene or an ethylene acetate or acrylate copolymer.

4. The use according to claim 1, 2 or 3 wherein the film is in the form of a laminate with one or more other elements.

5. The use according to any one of claims 1 to 4 wherein the article produced comprises a plastic bag, bottle, pouch, tray or tub comprising the film.

6. The use according to any one of claims 1 to 5 wherein the article is produced simultaneously with film formation from said blend.

7. The use according to any one of claims 1 to 4 wherein the article produced comprises a container or package formed from the film and having a paper lid or section, or comprises a window or wall formed from the film.

8.     Articles produced by the film use according to any one of claims 1 to 7 when in sterilized form.

9.     An article having at least one major portion thereof being a heat sealed section of a thermoplastic resin member, said member having a thickness in the range of about 0.25 to about 35 mils and having a combination of physical properties comprising tensile strength in the range of 2,000 to 7,000 psi, elongation at break of at least 400 percent, stiffness in the range of 15,000 to 100,000 psi and tear strength measured in the machine direction of 40 to 600 g/mil, said member comprising a compatible blend of (a) a first member selected from polypropylene resins, (b) a second member selected from ethylene propylene elastomers, and (c) a third member selected from polyethylene resins, the weight ratio of said members being in the range of at least 20% for said first member, 10% to 60% for said second member, and 10% to 60% for said third member.

10.    A plastic bag structure having at least one major portion thereof being a heat sealed section of thermoplastic resin film, said film having a thickness in the range of about 0.25 to about 35 mils and having a combination of physical properties comprising tensile strength in the range of 2,000 to 7,000 psi, elongation at break of at least 400 percent, stiffness in the range of 15,000 to 100,000 psi and tear strength measured in the machine direction in the range of 40 to 600 g/mil, said film comprising a compatible blend of (a) a first member selected from polypropylene resins, (b) a second member selected from ethylene propylene elastomers, and (c) a third member selected from polyethylene resins, the weight ratio of said members being in the range of at least 20% for said first member, 10% to 60% for said second member, and 10% to 60% for said third member.

11.     The structure of Claim 9 or 10 wherein the percent by weight range for the members are:

    first member - 30 - 70%
    second member - 20 - 50%
    third member - 10 - 50%

based on total weight of the resins.

12.     In a sterilized package comprising a package having a compartment containing sterilized contents, the improvement comprising having at least a major portion of said compartment formed from a thermoplastic film element, said film element having a thickness in the range of about 0.25 to 35 mils and having a combination of physical properties comprising tensile strength in the range of 2,000 to 7,000 psi, elongation at break of at least 400 percent, stiffness in the range of 15,000 to 100,000 psi, said element being composed of a compatible blend of (a) a first member selected from polypropylene resins, (b) a second member selected from ethylene propylene elastomers, and (c) a third member selected from polyethylene resins, said members being in the range of at least 20% for said first member, 10% to 60% for said second member, and 10% to 60% for said third member.

13.     In a process of sterilization of packages wherein a container is filled with separate contents, the container is sealed and the container is subjected to sterilization by heat, the improvement comprising having at least a major portion of said container formed from a thermoplastic resin element, said element having a thickness in the range of about 0.25 to 35 mils and having a combination of physical properties comprising tensile strength in the range of 2,000 to 7,000 psi, elongation at break of at least 400 percent, stiffness in the range of 15,000 to 100,000 psi, said element being composed of a compatible blend of (a) a first member selected from polypropylene resins, (b) a second member selected from ethylene-propylene elastomers, and (c) a third member selected from polyethylene resins, said members being in the range of at least 20% for said first member, 10% to 60% for said second member, and 10% to 60% for said third member.

14.    An article having at least one major portion thereof being a heat sealed section of thermoplastic resin, said resin when in film form of a thickness in the range of about 0.25 to about 35 mils having a combination of physical properties comprising tensile strength in the range of 2,000 to 7,000 psi, elongation at break of at least 400 percent, stiffness in the range of 15,000 to 100,000 psi and tear strength measured in the machine direction of 40 to 600 g/mil, said resin comprising a compatible blend of (a) a first member selected from polypropylene resins, (b) a second member selected from ethylene propylene elastomers, and (c) a third member selected from polyethylene resins, the weight ratio of said members being in the range of at least 20% for said first member, 10% to 60% for said second member, and 10% to 60% for said third member.

15.    In a process of sterilization of packages wherein a container is filled with separate contents, the container is sealed and the container is subjected to sterilization by radiation, the improvement comprising having at least a major element of said container formed from a thermoplastic resin, said element having a thickness in the range of about 0.25 to 35 mils and having a combination of physical properties comprising tensile strength in the range of 2,000 to 7,000 psi, elongation at break of at least 400 percent, stiffness in the range of 15,000 to 100,000 psi, said resin being composed of a compatible blend of (a) a first member selected from polypropylene resins, (b) a second member selected from ethylene-propylene elastomers, and (c) a third member selected from polyethylene resins, said members being in the range of at least 20% for said first member, 10% to 60% for said second member, and 10% to 60% for said third member.

FIG.1

2 ;

FIG.2